# FASCICULE DE BREVET EUROPEEN

(11) **EP 1 131 048 B1**
(45) Date de publication et mention de la délivrance du brevet: **26.02.2003**
(21) Numéro de dépôt: 99972914.8
(22) Date de dépôt: 26.11.1999
(51) Int. Cl.: A61K 7/48, A61K 35/78

(54) **EXTRAIT DE TOURTEAU DE GRAINES DE NOIX**
WALNUSSSAMENSCHROTMEHLEXTRAKT
WALNUT SEED MEAL EXTRACT

(30) Priorité: 27.11.1998 FR 9815162
(43) Date de publication de la demande: 12.09.2001
(73) Titulaire: GATTEFOSSE S.A., 69800 Saint Priest (FR)
(72) Inventeur: LAFORET, Jean-Pierre, F-69740 Genas (FR)
(74) Mandataire: Vuillermoz, Bruno
(86) Numéro de dépôt international: FR9902925
(87) Numéro de publication internationale: WO00032163

(56) Documents cités:
- WO-A-91/11169
- FR-A- 2 207 652
- GB-A- 673 501
- STN, Serveur de Bases de Données, Karlsruhe, DE, Fichier Chemical Abstracts, Vol 103, AN=21432 * résumé * XP002115327

## Description

L'invention se rapporte à un extrait de tourteau de graine de noix. Elle concerne également une composition cosmétique comprenant ledit extrait. Elle a aussi pour objet différentes applications de l'extrait et donc de la composition cosmétique.

Dans la suite de la description et dans les revendications, on désigne par « tourteau de graine de noix », le résidu de pressage de la graine de la noix après extraction de l'huile qu'elle contient communément appelée « huile de noix ».

Les noyers appartenant à la famille des juglandacées sont largement répandus et cultivés dans les pays tempérés. Il s'agit notamment du Juglans regia présent en Europe ou encore du Juglans nigra présent en Amérique, lesquels font l'objet d'un certain nombre d'applications.

On a ainsi décrit, dans le document RO-A-103 270, une composition cosmétique utilisée pour le lavage des cheveux et comprenant en combinaison un salicylate et un extrait alcoolique de noyer du type Juglans regia. Toutefois, rien n'est indiqué concernant la partie de l'arbre à partir de laquelle l'extrait est obtenu.

On a également décrit, dans le document EP-A-0 306 853, une composition cosmétique présentant des propriétés germicides contenant des extraits d'écorces de racines d'arbres de la famille des juglandacées.

De même, on a identifié certaines parties de la noix comme présentant des propriétés intéressantes.

C'est par exemple le cas du brou de noix constituant la partie charnue externe de la noix, lequel contient un composé naphtoquinonique (le juglone) utilisé comme teinture capillaire.

En revanche, le résidu de pressage de la graine de la noix constituant le tourteau n'a jamais été valorisé et n'a par conséquent pas reçu d'application particulière.

Le Demandeur a cherché à isoler un extrait de tourteau de graine de noix et a constaté qu'il présentait un certain nombre de propriétés intéressantes.

L'invention concerne donc un extrait de tourteau de graine de noix produites par un noyer du genre Juglans susceptible d'être obtenu par une étape de macération aqueuse dudit tourteau, puis une étape de concentration.

Avantageusement, la concentration est effectuée par osmose inverse.

Par ailleurs et en pratique, la macération est effectuée à une température comprise entre 3 et 10° C, avantageusement 4° C. Une température inférieure à 4°C est trop froide et peut provoquer la formation de glace. Une température supérieure à 4° C est au contraire trop élevée et risque de provoquer la dégradation de l'activité de l'extrait.

L'extrait obtenu se présente sous forme d'une solution aqueuse concentrée.

Pour obtenir l'extrait sous forme de poudre, on fait suivre l'étape de concentration par une étape de séchage ou de lyophilisation.

Avantageusement, les noix dont est issu l'extrait de tourteau sont produites par un noyer commun (Juglans regia).

Comme déjà dit, l'extrait de tourteau de noix de l'invention présente un certain nombre de propriétés intéressantes de sorte qu'il peut être utilisé pour différentes applications, notamment au sein de compositions cosmétiques.

### 1/ Activité protectrice de l'extrait de tourteau de graine de noix vis-à-vis de l'oxydation intracellulaire provoquée par un stress oxydatif du type UVB

L'approche expérimentale utilisée pour cette évaluation est basée sur la mesure du taux d'oxydation intracellulaire, après exposition aux UVB de keratinocytes humains cultivés en absence et en présence de l'extrait de l'invention.

### Méthodes

Des kératinocytes humains normaux isolés à partir de peaux de prépuces obtenues lors d'interventions chirurgicales sont cultivés en milieu KGM "serum free" à 37° C, en atmosphère humide air-CO₂ (95-5 %).

Après étude de cytotoxicité, trois concentrations d'extrait ont été retenues pour l'évaluation : 10 µg/ml, 100 µg/ml, 250 µg/ml.

### Principe du test

Le principe du test est basé sur la mesure du degré d'oxydation intracellulaire à l'aide d'une sonde spécifique : DCFH-DA (2', 7' - trichlorodihydrofluoresceïne diacétate).

Le DCFH-DA, sonde non fluorescente, pénètre par diffusion passive à l'intérieur des cellules. Après clivage des groupements acétate par les estérases intracellulaires, le DCFH s'accumule au niveau du cytosol. L'oxydation intracellulaire du DCFH par différentes Espèces Oxygénées Réactives (EOR) conduit à la formation d'un produit fluorescent.

La mesure des intensités de fluorescence permet d'évaluer le degré d'oxydation des cellules soumises à un stress oxydatif.

Après avoir été détachées de leur support, les suspensions de keratinocytes sont ensemencées dans des plaques 24 cupules. Les cellules sont incubées à 37° C pendant 24 heures, avec le milieu contenant l'extrait à l'étude.

Après incubation avec les différentes concentrations d'extrait à tester, les cultures sont rincées à l'aide d'une solution de PBS, puis exposées aux UVB à travers une solution de PBS.

Après irradiation des cellules, la solution de PBS est remplacée par une solution de DCFH-DA. Après incubation de la sonde fluorescente, les tapis cellulaires sont abondamment rincés au PBS, puis les cellules sont remises à incuber à 37° C pendant 24 heures avec du milieu de culture neuf.

En fin d'essai, les kératinocytes mis en suspension sont transférés dans des cuves thermostatées et les intensités de fluorescence (Abs = 502 nm, Em = 520 nm) sont mesurées et exprimées en pourcentage par rapport aux cultures contrôles non irradiées.

Les résultats sont exprimés dans le tableau suivant :

| | **Degré d'oxydation** | **Activité protectrice** |
|---|---|---|
| **Témoin** | | |
| - pas UV | 100 | - |
| - 25 mJ/cm² | 144 | - |
| - 50 mJ/cm² | 176 | - |

| **10 µ**g**/ml** | | |
|---|---|---|
| - pas UV | 100 | - |
| - 25 mJ/cm² | 116 | 39 |
| - 50 mJ/cm² | 125 | 65 |

| **100 µ**g**/ml** | - | |
|---|---|---|
| - pas UV | 100 | - |
| - 25 mJ/cm² | 107 | 74 |
| - 50 mJ/cm² | 115 | 79 |

| **250 µ**g**/ml** | | |
|---|---|---|
| - pas UV | 100 | - - |
| - 25 mJ/cm² | 98 | 104 |
| - 50 mJ/cm² | 106 | 92 |

Dans les conditions de cette étude, l'extrait de l'invention protège de manière dose dépendante la cellule vis-à-vis du stress oxydatif induit par irradiation UVB. L'extrait est donc capable de réguler le taux d'espèces oxygénées réactives (EOR) qui se forment. L'extrait possède la capacité de protéger la cellule du stress oxydatif, c'est-à-dire de bloquer la formation de EOR et/ou d'inhiber leur réactivité par un processus de "piégeage".

Dans une première application, l'extrait de l'invention peut donc être utilisé pour protéger les cellules cutanées du stress oxydatif induit par irradiation UVB.

### 2/ Activité protectrice des cellules vis-à-vis de l'apoptose induite par les radiations UVB

Les EOR capables de réagir directement avec l'ADN peuvent introduire au niveau de ce constituant de multiples modifications chimiques. Ces modifications, qui ont pour conséquence une perturbation du programme génétique de la cellule, sont dans l'ensemble corrigées par des enzymes dites de réparation.

Toutefois, lorsque cette réparation n'est pas totale, la cellule rentre dans un programme de mort cellulaire programmée ou apoptose conduisant à leur élimination.

### Evaluation de l'activité protectrice des cellules vis-à-vis de l'apoptose induite par les radiations UVB

Ce pouvoir protecteur vis-à-vis de l'apoptose induite par les UVB a été évalué sur cultures de keratinocytes monocouches, par détermination du pourcentage de cellules apoptotiques au moyen du kit APOPTAG ® (test semi-quantitatif).

### Méthode

Les keratinocytes sont obtenus à partir de cultures primaires de prépuces et sont mis à proliférer dans un milieu conditionné (KGM) "serum free".

Après avoir été détachées de leur support, les suspensions de keratinocytes sont mises en culture dans des plaques 24 puits. Les cellules sont cultivées dans du milieu Iscove complémenté par des antibiotiques et du sérum de veau foetal (5 %). Elles sont mises en présence de l'extrait de l'invention pendant 24 heures à deux concentrations (10 et 100 µg/ml), puis stimulées par des radiations UVB.

Le nombre de cellules apoptotiques est déterminé 72 heures après irradiation en milieux traités et non traités en utilisant le kit APOPTAG ®. Les cassures d'ADN sont visualisées par émission de fluorescence. Le nombre de cellules en apoptose est évalué par comptage du nombre de cellules fluorescentes sur un total de 200 cellules.

Le pourcentage de réduction des cellules apoptotiques sous l'action de l'extrait de l'invention est déterminé.

Les résultats sont exprimés dans le tableau suivant.

| **Extrait de l'invention (µ**g**/ml)** | **Pourcentage de cellules apoptotiques induit par les UV** |
|---|---|
| 0 | 70 |
| 10 | 55 |
| 100 | 49,5 |

Sous l'influence de l'extrait de l'invention, le taux de cellules apoptotiques engendré par les UV (70 %) est réduit de manière dose dépendante.

Les résultats de cette étude démontrent que l'extrait de l'invention possède des propriétés antiapoptotiques à faibles concentrations, consécutives à sa capacité à limiter la formation et/ou l'action des EOR.

### 3/ Activité anti-inflammatoire de l'extrait de tourteau de graine de noix de l'invention

In vivo, les stress environnementaux et notamment les UV, en stimulant la formation de radicaux libres, favorisent l'expression de nombreux médiateurs épidermiques, capables de conditionner le développement d'une inflammation.

Le Demandeur a constaté que l'extrait de tourteau de graine de noix était efficace pour limiter le développement de la réaction inflammatoire.

Cette activité a été évaluée in vitro sur des kératinocytes et des macrophages.

Les kératinocytes sont obtenus à partir de cultures primaires de prépuces et sont mis à proliférer dans un milieu conditionné pendant deux semaines. Au terme de cette période, les kératinocytes sont mis en culture dans des plaques 24 puits.

Les macrophages sont obtenus à partir de prélèvements sanguins après centrifugation. Après traitement usuel, ils sont mis à adhérer sur boite de Pétri.

On teste l'extrait de l'invention à une concentration déterminée. Un lot de culture témoin, non traité par l'extrait de l'invention est mené en parallèle.

Les kératinocytes et les macrophages obtenus sont ensuite soumis aux deux stimuli suivants.

Le premier stimulus consiste à déclencher une réaction inflammatoire spécifique en activant les kératinocytes et les macrophages par de l'IL₄ (10 nanogrammes par millilitre) pendant 48 heures, afin d'induire la production de CD23 (récepteur de basse affinité pour les IgE), lesquels sont ensuite activés par les complexes immuns à IgE.

Le second stimulus consiste à déclencher dans les cellules une réaction inflammatoire non spécifique en activant les kératinocytes et les macrophages par un mélange IFN γ (1000 unités par millilitre) et d'extrait de lipopolysaccharide LPS (10 microgrammes par millilitre).

Les cellules ainsi stimulées sont maintenues en culture pendant 48 heures avant de prélever les surnageants qui sont ensuite testés par colorimétrie ou méthode ELISA pour déterminer leur contenu respectif en dérivés nitrés (NO) et en TNFα, constituant les médiateurs pro-inflammatoires.

Les résultats du test sont présentés dans le tableau ci-dessous.

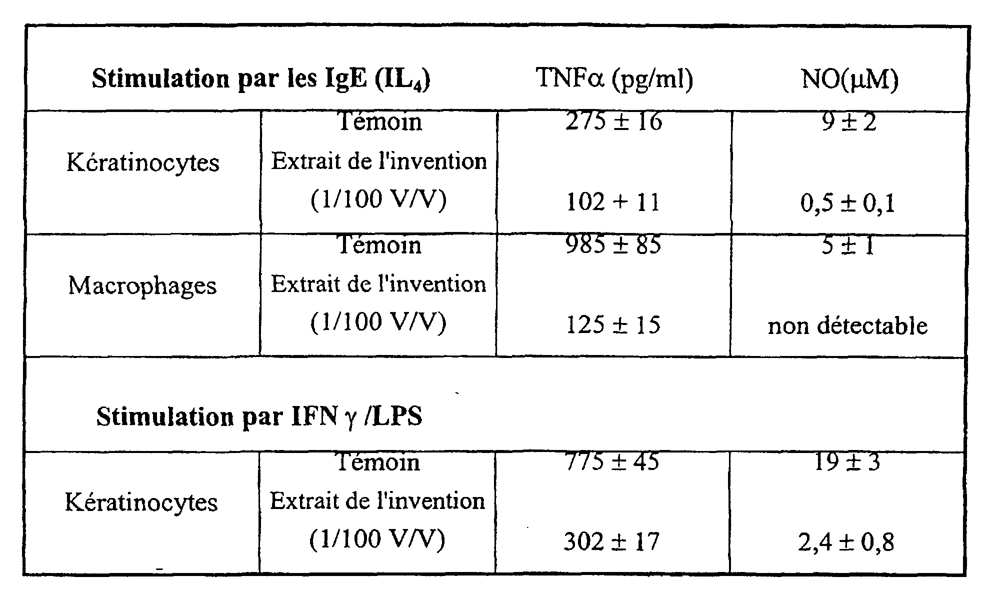

Les résultats de ce test démontrent la capacité de l'extrait de l'invention à moduler la libération des médiateurs pro-inflammatoires en réponse à divers stimuli.

En effet, dans le cas d'une inflammation dépendante des IgE, déclenchée par IL₄, l'extrait de l'invention présente une activité anti-inflammatoire sur les kératinocytes (diminution de la synthèse de NO et de TNFα). On- note une activité renforcée du même type sur les macrophages.

Dans le cas d'une inflammation non spécifique déclenchée par l'association IFNγ/LPS, seuls les kératinocytes sont à même de produire à la fois des TNFα et des dérivés nitrés. Dans ce cas précis, l'extrait de l'invention présente également une activité anti-inflammatoire significative.

L'extrait de l'invention pourra être avantageusement incorporé dans des compositions cosmétiques destinées à être appliquées sur des peaux sensibles, qui sont des peaux hyper-réactives et donc très réceptives aux stress environnementaux, tels que les radiations UV, les agents chimiques irritants, les chocs thermiques, la pollution, les agents allergiques.

Dans ce cas, on pourra protéger la peau de tous stress environnemental et notamment du rayonnement UV selon un procédé consistant à appliquer sur la peau une quantité efficace de l'extrait de l'invention ou d'une composition comprenant ledit extrait.

L'extrait de l'invention peut également être incorporé dans des compositions cosmétiques ayant un potentiel irritant comme celles comprenant des agents tensioactifs ou des α-hydroxyacides, afin de limiter l'impact de certaines réactions proinflammatoires liées à ces composants.

### 4/ Activité anti-âge de l'extrait de tourteau de graine de noix de l'invention vis à vis de la peau

On a également constaté que l'extrait de tourteau de graine de noix de l'invention était efficace dans la lutte contre le vieillissement de la peau de par son activité :
- stimulante de la synthèse protéique des kératinocytes de l'épiderme et fibroblastes du derme ;
- anti-collagènasique ;
- anti-élastasique.

L'extrait peut donc être utilisé dans des compositions cosmétiques destinées à lutter contre le vieillissement de la peau.

Le vieillissement de la peau résulte d'une sénescence programmée conduisant à une atrophie du tissu cutané, qui apparaît particulièrement prononcé au niveau du derme. Cette atrophie provient d'un ralentissement du métabolisme cellulaire et est à l'origine de l'apparition notamment de rides.

Le derme est un tissu conjonctif composé d'une matrice extracellulaire (MEC) synthétisée par les fibroblastes. La MEC, responsable des propriétés mécaniques de la peau, est constituée de différentes protéines, dont le collagène (type I et type III) l'élastine et les glycosaminoglycanes (essentiellement acide hyaluronique et dermatane sulfate).

Au cours du temps, s'instaure une altération autant qualitative que quantitative de la matrice extra-cellulaire. Cette altération se traduit par une dégénérescence du réseau collagénique, du réseau élastique et par une diminution de la teneur en glycosaminoglycanes et plus particulièrement en acide hyaluronique. Ces modifications résultent à la fois d'une diminution de la capacité des fibroblastes à synthétiser la matrice extracellulaire, et d'un déséquilibre dans l'expression de certaines protéinases, notamment les protéinases dites Matrix Métallo protéinases et les proteinases dites Tissu Inhibitor Métallo Protéinases.

De la sorte, au cours du vieillissement, les propriétés mécaniques de la peau régressent et on observe une diminution de la force de tension ou de la rigidité (perte du réseau collagènique), une diminution de l'élasticité et de la résilience (dégénérescence du réseau élastique), le tout accompagné d'un effondrement de l'hydradatation (baisse du taux d'acide hyaluronique) à l'origine d'une perte de la turgescence dermique.

Des tests ont été réalisés pour démontrer l'activité anti-âge de l'extrait de l'invention vis à vis de la peau.

### • activité stimulante de l'extrait de l'invention sur la synthèse protéique des kératinocytes de l'épiderme et des fibroblastes du derme humain

### a) Cytotoxicité

On a tout d'abord réalisé un essai de cytotoxicité de l'extrait de l'invention sur des fibroblastes pour déterminer la dose maximale d'extrait n'entraînant pas la cytotoxicité.

La viabilité cellulaire est évaluée, en fin d'essai, par un test colorimétrique au MTT. Le principe de ce test résulte de la transformation, par la succinyl déshydrogénase mitochondriale des cellules métaboliquement actives, du 3(4,5-diméthylthiazol-2-yl)-2,5 diphényl tetrazolium bromide (substrat soluble jaune à l'état oxydé) en formazan bleu-violet. La densité optique de la solution bleu-violet obtenue en fin d'essai est proportionnelle au nombre de cellules vivantes.

On a évalué la cytotoxicité de l'extrait de l'invention à partir de neuf concentrations différentes d'extrait comprises entre 0,01 et 10 mg/ml. Une première évaluation a été effectuée après 24 heures.

On a réalisé une seconde évaluation après 48 heures sur des concentrations comprises entre 0,05 et 5 mg/ml.

Les résultats figurent dans le tableau ci-après.

| Concentration mg/ml | 0,1 | 0,25 | 0,5 | 0,75 | 1 | 2,5 | 5 | 10 |
|---|---|---|---|---|---|---|---|---|
| Viabilité (24 h) | 108 % | non déterminé | 112 % | non déterminé | 107 % | non déterminé | 80 % | 68 % |
| Viabilité (48 h) | 110 % | 109 % | 107 % | 99 % | 94 % | 77 % | 53 % | non déterminé |

Après 24 heures de contact, les concentrations d'extrait inférieures ou égales à 1 mg/ml n'induisent aucune diminution significative de la réponse cellulaire vis-à-vis du MTT. De même, les résultats après 48 heures confirment la non toxicité des concentrations inférieures ou égales à 1 mg/ml. De la sorte, la concentration en extrait de tourteau de graine de noix de 1 mg/ml a été retenue comme dose maximale non cytotoxique pour la suite de cette étude.

### b) Etude sur les kératinocytes de l'épiderme

On cultive des kératinocytes en monocouches. A J-1, les kératinocytes sont détachés de leur support par trypsination douce. Après centrifugation, les cellules sont remises en suspension dans un milieu de culture optimal. Les suspensions cellulaires sont ensuite ensemencées.

24 heures après ensemencement (Jₒ), les milieux de culture sont éliminés et remplacés par un milieu contenant différentes concentrations d'extrait de tourteau de graine de noix.

Une fois traitées, les kératinocytes sont replacés à l'étuve à 37° C et incubés pendant 72 heures en atmosphère air-CO₂ (95/5 %).

On réalise en fin d'essai un dosage de protéines cellulaires totales par la méthode au bleu de Cooomassie. Ce test consiste tout d'abord à rincer en fin de traitement les cellules avec du PBS. On ajoute ensuite une solution de NaOH (50 µl) dans chaque puits. Après 10 minutes d'incubation, 200 µl d'une solution diluée de Biorad est ajoutée dans chacun des puits. On mesure ensuite l'absorbance des solutions à 570 nanomètres après cinq minutes d'incubation. On construit parallèlement une gamme étalon à l'aide de BSA (Bovine Serum Albumin) permettant de transformer les densités optiques obtenues en équivalents microgrammes de protéines par puits.

Diverses concentrations d'extrait de tourteau de noix ont ainsi été testées respectivement 0,1 mg/ml et 0,25 mg/ml dans le milieu suboptimal. On a également réalisé un lot témoin suboptimal et un lot témoin optimal.

Le dosage des protéines s'est fait à J₀ et à J₃. On a reproduit les résultats dans le tableau ci-dessous.

| | Protéines (µg/puits) | |
|---|---|---|
| | J₀ | J₃ |
| Témoin optimal | 3,04 | 17,70 |
| Témoin suboptimal | 3,04 | 13,93 |
| Concentration 0,1 mg/ml | 3,04 | 19,81 |
| Concentration 0,25 mg/ml | 3,04 | 19,33 |

On observe une très nette augmentation de la masse de protéines cellulaires au niveau des kératinocytes traitées avec l'extrait de l'invention.

De la sorte, l'extrait de l'invention a donc bien un effet stimulant du métabolisme cellulaire, c'est-à-dire de la synthèse protéique des kératinocytes.

### c) Etude sur les fibroblastes

On a étudié dans ce test les effets de l'extrait de l'invention sur l'incorporation de leucine dans différentes fractions protéiques néosynthétisées par des fibroblastes humains en culture monocouche.

Après culture des fibroblastes dermiques humains jusqu'à confluence des cellules, on change le milieu de culture par un milieu contenant 2 % de SVF (Sérum de Veau Foetal) avec :
- soit l'extrait de l'invention à 4 concentrations différentes respectivement 0,25 mg/ml, 0,1 mg/ml, 0,05 mg/ml et 0,01 mg/ml ;
- soit de la vitamine C (produit de référence).

Un lot de culture témoin non traité est mené en parallèle.

Après 24 heures d'incubation à 37° C, on ajoute de la leucine marquée (³H leucine) dans le milieu de culture et on laisse incuber 48 heures supplémentaires. On recueille séparément :
- d'une part, le milieu de culture,
- d'autre part, le lysat des cellules après lyse cellulaire,
- et enfin la fraction insoluble (membranes, matrice déposée).

Les macromolécules sont extraites puis on mesure par scintillation liquide l'incorporation du précurseur radioactif de ces molécules.

Les résultats figurent dans le tableau ci-dessous.

| **Macromolécules extraites du milieu de culture** | | | |
|---|---|---|---|
| **Traitement** | **coups/minute** | **% Témoin** | **P** (significativité) |
| Témoin | 26257 ± 3567 | 100 | - |
| Vitamine C | 34748 ± 2599 | 132 | < 0,05 |
| Extrait de l'invention | | | |
| 0,25 mg/ml | 34088 ± 1971 | 130 | < 0,05 |
| 0,1 mg/ml | 32572 ± 4184 | 124 | > 0,05 |
| 0,05 mg/ml | 28512 ± 2771 | 109 | > 0,05 |
| 0,01 mg/ml | 27664 ± 2519 | 105 | > 0,05 |

| **Macromolécules extraites du milieu intracellulaire** | | | |
|---|---|---|---|
| **Traitement** | **coups/minute** | **% Témoin** | **P** (significativité) |
| Témoin | 37298 ± 2213 | 100 | - |
| Vitamine C | 35953 ± 4349 | 96 | > 0,05 |
| Extrait de l'invention | | | |
| 0,25 mg/ml | 42786 ± 5990 | 115 | > 0,05 |
| 0,1 mg/ml | 43571 ± 3921 | 117 | > 0,05 |
| 0,05 mg/ml | 41530 ± 2844 | 111 | > 0,05 |
| 0,01 mg/ml | 45199 ± 1675 | 121 | > 0,05 |

| **Fraction insoluble (matrice, membranes)** | | | |
|---|---|---|---|
| **Traitement** | **coups/minute** | **% Témoin** | **P** (significativité) |
| Témoin | 50372 ± 7511 | 100 | - |
| Vitamine C | 56865 ± 3853 | 113 | > 0,05 |
| Extrait de l'invention | | | |
| 0,25 mg/ml | 65854 ± 5154 | 131 | < 0,01 |
| 0,1 mg/ml | 64060 ± 983 | 127 | < 0,05 |
| 0,05 mg/ml | 54621 ± 5542 | 108 | > 0,05 |
| 0,01 mg/ml | 56352 ± 86 | 112 | > 0,05 |

| **Total** | | | |
|---|---|---|---|
| **Traitement** | **coups/minute** | **% Témoin** | **P** (significativité) |
| Témoin | 113928 ± 12162 | 100 | - |
| Vitamine C | 127566 ± 10289 | 112 | > 0,05 |
| Extrait de l'invention | | | |
| 0,25 mg/ml | 142737 ± 5090 | 125 | < 0,01 |
| 0,1 mg/ml | 140204 ± 8253 | 123 | < 0,01 |
| 0,05 mg/ml | 124663 ± 7706 | 109 | > 0,05 |
| 0,01 mg/ml | 129216 ± 2953 | 113 | > 0,05 |

On constate que l'extrait à une concentration de 0,25 mg/ml a stimulé de façon significative l'incorporation de leucine dans la fraction protéines sécrétées solubles (identique au produit de référence vitamine C).

On observe également qu'à une concentration de 0, 1 mg/ml, l'extrait stimule encore l'incorporation de leucine dans cette fraction.

De même, l'activité stimulante de l'extrait de l'invention à 0,25 mg/ml et 0,1 mg/ml a été observée dans la fraction protéines insolubles.

En revanche, on note que l'extrait de l'invention ne modifie pas significativement l'incorporation de leucine dans la fraction cellulaire soluble, ce qui confirme que l'extrait de stimule pas la multiplication des fibroblastes en culture.

En conclusion, l'extrait de l'invention stimulant l'incorporation de leucine par les fibroblastes, on en déduit que l'extrait augmente significativement la synthèse des protéines et plus spécifiquement les protéines sécrétées par les fibroblastes, à savoir les protéines de la matrice extracellulaire (collagènes, glycosaminoglycanes).

### • activité anti-élastasique de l'extrait de l'invention

Cette activité est évaluée sur des extraits cellulaires de fibroblastes de derme humain, isolés à partir de peau de prépuce et cultivés selon les techniques de routine à 37° C, en atmosphère humide air-CO₂ (95/5 %).

Les fibroblastes sont cultivés en milieu DMEM supplémenté en sérum de veau foetal (SVF 10 %) et passés régulièrement jusqu'à obtention d'une biomasse suffisante.

Pour réaliser le test, les cellules sont décollées de leur support par trypsination. Après centrifugation et traitement adéquat, on récupère les extraits cellulaires.

L'activité élastase des extraits cellulaires a été évaluée en utilisant comme substrat le Suc-(L-Ala)₃-p Nitroanilide (SANA). Des aliquots de l'extrait cellulaire sont pré-incubés à 37° C dans du tampon TEA (triethanolamine) à pH = 7,8, seul ou dans du tampon contenant différentes concentrations d'extraits.

Après incubation, une solution de SANA est ajoutée au mélange réactionnel. L'activité catalytique de l'extrait cellulaire est appréciée en mesurant le taux de libération de p Nitroanilide, lequel présente un maximum d'absorption à 405 nm.

Les densités optiques à 405 nm sont enregistrées pendant 1 heure 30 à 2 heures.

Les effets de l'extrait de l'invention sur l'activité élastasique ont été étudiés pour cinq concentrations différentes, respectivement 0,5 mg/ml, 1 mg/ml, 2 mg/ml, 5 mg/ml et 10 mg/ml. Un contrôle positif a été introduit dans l'essai (dichloroisocoumarine à 2 mM).

On a regroupé les résultats dans le tableau suivant.

| | Témoin | 0,5 | 1 | 2 | 5 | 10 | DCI |
|---|---|---|---|---|---|---|---|
| mU elastase/10⁶ cell | 0,682 | 0,577 | 0,503 | 0,424 | 0,332 | 0,310 | 0,431 |
| Inhibition | - | 15,3 | 26,3 | 37,7 | 51,4 | 54,7 | 37 |

Les résultats obtenus montrent que l'cxtrait de l'invention est capable d'inhiber de manière dose dépendante l'activité élastasique.

Dans les conditions expérimentales retenues, la concentration inhibitrice 50 de l'extrait a été évaluée à 5,6 mg/ml.

### • -activité anti-collagènasique de l'extrait de l'invention

On a étudié l'effet de l'extrait de l'invention sur l'activité anticollagènase.

Cette activité a été évaluée par la méthode de la fluorescamine.

Le test est basé sur la formation de composés fluorescents entre la fluorescamine et les amines primaires, les acides aminés et les peptides. En pratique, on mesure l'augmentation de l'émission de fluorescence obtenue après incubation d'une solution de collagène de type I et de collagènase en présence de fluorescamine.

L'évaluation de l'activité catalytique de la collagènase se fait en absence ou en présence de différentes concentrations de l'extrait, respectivement pour 0,5 mg/ml, 1 mg/ml, 2 mg/ml, 5 mg/ml et 10 mg/ml.

Les résultats sont reproduits dans le tableau ci-après.

| **Concentration** **mg/ml** | **Inhibition** |
|---|---|
| 0,5 | 6,4 % |
| 1 | 6,6% |
| 2 | 12,6% |
| 5 | 19,5% |
| 10 | 17,2% |

Comme le montrent les résultats, on observe un effet inhibiteur de l'activité collagènasique.

Comme déjà dit, l'invention concerne également une composition cosmétique comprenant un extrait de tourteau de graine de noix tel que décrit précédemment.

Pour obtenir une composition cosmétique présentant les propriétés précitées, l'extrait de tourteau de l'invention est utilisé sous forme d'une solution de concentration comprise entre 10 et 40 grammes de matière sèche par litre de solvant, avantageusement 30 g/l.

En pratique, la composition contient entre 0,5 et 10 %, avantageusement entre 2 et 5 % en poids de la solution d'extrait de l'invention précitée de tourteau de graine de noix. Bien entendu, l'extrait sera incorporé dans la composition cosmétique avec tout excipient usuel de formulation.

La composition cosmétique pourra notamment se présenter sous forme de crème, lait, gel, sérums, microémulsions ou autre.

Comme déjà dit, cette composition pourra être utilisée en cosmétique pour le traitement du vieillissement cutané ou encore comme protecteur de la peau contre les stress environnementaux, induits notamment par les rayonnements UV.

L'invention concerne enfin un procédé de traitement cosmétique de lutte contre le vieillissement de la peau, caractérisé par le fait qu'il consiste à appliquer sur la peau une quantité efficace de la composition cosmétique décrite ci-avant.

Les avantages de l'invention ressortiront mieux des exemples de réalisation qui suivent.

### Exemple 1

### Formulation d'une crème protectrice à base de l'extrait de l'invention

On prépare les cinq phases suivantes :

| Phase I | |
|---|---|
| Plurol ® stéarique | 5 % |
| Alcool cétylique | 2 % |
| Myristate d'octyldodécyle | 3 % |
| Labrafac ® lipophile WL 1349 | 3 % |
| Ceraphyl 368 | 4 % |
| Dimethicone | 5 % |
| Huile de Camellia | 3 % |
| Phenonip | 0,5 % |

| Phase II | |
|---|---|
| Eau déminéralisée | 35 % |
| Avicel RC 591 | 1 % |

| Phase III | |
|---|---|
| Eau déminéralisée | qsp |
| Ultrez 10 | 0,15 % |
| Hydroxyde de sodium (10 % solution) | 0,3 % |

| Phase IV | |
|---|---|
| Dow corning 1403 | 2 % |

| Phase V | |
|---|---|
| Solution d'extrait de l'invention (30 g/l) | 3 % |

La fabrication de la crème est réalisée selon le procédé suivant.

On disperse tout d'abord l'Avicel RC 591 dans l'eau. On saupoudre ensuite l'Ultrez 10 dans l'eau. On neutralise avec l'hydroxyde de sodium. On ajoute alors sous agitation les phases II et III chauffées préalablement à 55° C dans la phase I chauffée à 75° C. On refroidit sous agitation le mélange et vers 50° C, on ajoute la phase IV. Sous agitation, on ajoute la phase V à une température voisine de 35° C.

### Exemple 2

### Formulation d'une crème anti-âge

| Phase I | |
|---|---|
| Apifil ® | 8 % |
| MOD | 10 % |
| ISIS | 10 % |
| Phenonip | 0,6 % |

| Phase II | |
|---|---|
| Eau déminéralisée - | qsp |
| Carbopol 934 | 0,2 % |

| Phase III | |
|---|---|
| Hydroxyde de sodium (10 % solution) | 0,4 % |

| Phase IV | |
|---|---|
| Transcutol ® CG | 5 % |
| Solution d'extrait de l'invention (30 g/l) | 5 % |
| Parfum | qsp |

La fabrication de la crème anti-âge est réalisée selon le procédé suivant.

On disperse tout d'abord le Carbopol dans la phase II. On ajoute ensuite sous agitation la phase II chauffée à 75° C dans la phase I chauffée également à 75° C. On ajoute l'hydroxyde de sodium. Toujours sous agitation, on refroidit le mélange et à une température voisine de 30° C, on ajoute les autres constituants.

### Exemple 3

### Formulation d'un gel moussant pour peaux sensibles

| Phase I | |
|---|---|
| Oronal LCG | 20 % |
| Oronal BLD | 20 % |
| Amonyl 440 NI | 10 % |
| Oramide DL 200 | 1,8 % |
| Softcutol ® O | 1 % |
| Phenonip | 0,5 % |
| Parfum | 0,5 % |

| Phase II | |
|---|---|
| Eau déminéralisée | qsp |
| Solution d'extrait de l'invention (30 g/l) | 2 % |

| Phase III | |
|---|---|
| Acide citrique (50 % solution) | 0,07 % |

La fabrication du gel moussant est réalisée selon le procédé suivant.

A froid, on mélange tous les constituants de la phase I dans l'ordre de la formule. On ajoute ensuite les phases II et III sous agitation lente.

### Exemple 4

### Formulation d'un lait après-solaire

| Phase I | |
|---|---|
| Tefose ® 2000 | 7 % |
| Alcool cétylique | 2 % |
| Geleol | 1 % |
| ISIS | 5 % |
| MOD | 5 % |
| Diméthicone | 2 % |

| Phase II | |
|---|---|
| Eau déminéralisée | qsp |
| Carbopol 941 | 0,1 % |
| Solution d'extrait de l'invention (30 g/l) | 5 % |

| Phase III | |
|---|---|
| Hydroxyde de sodium (10 % solution) | 0,2 % |

| Phase IV | |
|---|---|
| Parfum | 0,2 % |
| Germaben II | 1 % |

Le lait après-solaire est fabriqué selon le procédé suivant.

On disperse tout d'abord le Carbopol dans l'eau. On ajoute ensuite sous agitation la phase II chauffée à 75° C dans la phase I chauffée à la même température. On incorpore ensuite la solution d'hydroxyde de sodium. Enfin, toujours sous agitation, on refroidit à une température voisine de 30° C et on ajoute les autres constituants.

Les avantages de l'invention ressortent bien de la description. On notera en particulier la capacité de l'extrait de tourteau de graine de noix à stimuler la synthèse protéique, notamment au sein des cellules du derme et de l'épiderme, de sorte à agir contre le vieillissement cutané. On note également son activité anti-inflammatoire, et protectrice vis-à-vis des "stress environnementaux" et notamment des UV engendrant des effets délétères cumulatifs.

Ces différentes applications font de l'extrait de tourteau de graine de noix un actif complet destiné à lutter non seulement contre le vieillissement intrinsèque en stimulant le métabolisme cellulaire, mais également extrinsèque par son activité protectrice.

## Revendications

1. Extrait de tourteau de graine de noix produites par un noyer du genre Juglans, susceptible d'être obtenu par une étape de macération aqueuse dudit tourteau puis une étape de concentration.

2. Extrait de tourteau de graine de noix selon la revendication 1 **caractérisé en ce que** l'étape de concentration est effectuée par osmose inverse.

3. Extrait de tourteau de graine de noix selon la revendication 1 **caractérisé en ce que** la macération est effectuée à 4° C.

4. Extrait de tourteau de graine de noix selon la revendication 1 **caractérisé en ce qu'**on fait suivre ladite étape de concentration par une étape de séchage ou de lyophilisation, afin d'obtenir une poudre.

5. Extrait de tourteau de graine de noix selon l'une des revendications 1 à 4, **caractérisé en ce que** la noix est produite par un noyer commun (Juglans regia).

6. Extrait de tourteau de graine de noix selon l'une des revendications 1 à 5 utilisé comme protecteur des cellules cutanées vis-à-vis du stress oxydatif induit par les UVB.

7. Extrait de tourteau de graine de noix selon l'une des revendications 1 à 5 utilisé comme agent anti-inflammatoire.

8. Extrait de tourteau de graine de noix selon l'une des revendications 1 à 5 utilisé comme agent anti-âge pour lutter contre le vieillissement de la peau.

9. Extrait de tourteau de graine de noix selon l'une des revendications 1 à 5 utilisé comme stimulateur de la synthèse des protéines par les cellules de l'épiderme.

10. Extrait de tourteau de graine de noix selon l'une des revendications 1 à 5 utilisé comme stimulateur de la synthèse des protéines (et notamment le collagène et les glysosaminoglycanes) par les cellules du derme.

11. Extrait de tourteau de graine de noix selon l'une des revendications 1 à 5 utilisé comme agent anti-collagènasique.

12. Extrait de tourteau de graine de noix selon l'une des revendications 1 à 5 utilisé comme agent anti-élastasique.

13. Extrait de tourteau de graine de noix selon l'une des revendications 1 à 5, utilisé pour son activité protectrice vis-à-vis de l'apoptose induite.

14. Composition cosmétique **caractérisée en ce qu'**elle comprend un extrait de tourteau de graine de noix selon l'une des revendications 1 à 5.

15. Composition cosmétique selon la revendication 14 **caractérisée en ce que** ledit extrait se présente sous forme d'une solution de concentration comprise entre 10 et 40 g de matière sèche par litre de solvant, avantageusement 30 g/l.

16. Composition cosmétique selon la revendication 15 **caractérisée en ce qu'**elle contient entre 0,5 et 10 %, avantageusement entre 2 et 5 % en poids de ladite solution.

17. Procédé de traitement cosmétique pour lutter contre le vieillissement de la peau **caractérisé en ce qu'**on applique sur la peau une quantité efficace de la composition cosmétique selon l'une des revendications 14 à 16.

## Claims

1. A seed cake extract of walnuts produced by a walnut tree of the genus Juglans which can be obtained by a step of aqueous maceration of said cake and then a concentration step.

2. The walnut seed cake extract as claimed in claim 1, **characterized in that** the concentration step is carried out by reverse osmosis.

3. The walnut seed cake extract as claimed in claim 1, **characterized in that** the maceration is carried out at 4°C.

4. The walnut seed cake extract as claimed in claim 1, **characterized in that** said concentration step is followed by a drying or freeze-drying step, so as to obtain a powder.

5. The walnut seed cake extract as claimed in one of claims 1 to 4, **characterized in that** the walnut is produced by a walnut tree (Juglans regia).

6. The walnut seed cake extract as claimed in one of claims 1 to 5, used as protecting agent for skin cells against oxidative stress induced by UVB radiation.

7. The walnut seed cake extract as claimed in one of claims 1 to 5, used as anti-inflammatory agent.

8. The walnut seed cake extract as claimed in one of claims 1 to 5, used as anti age agent for combating skin ageing.

9. The walnut seed cake extract as claimed in one of claims 1 to 5, used as stimulator of the synthesis of proteins by cells of the epidermis.

10. The walnut seed cake extract as claimed in one of claims 1 to 5, used as stimulator of the synthesis of proteins (and in particular collagen and glycosaminoglycans) by cells of the dermis.

11. The walnut seed cake extract as claimed in one of claims 1 to 5, used as anti collagenase agent.

12. The walnut seed cake extract as claimed in one of claims 1 to 5, used as anti elastase agent.

13. The walnut seed cake extract as claimed in one of claims 1 to 5, used for its protective activity against induced apoptosis.

14. A cosmetic composition, **characterized in that** it comprises the walnut seed cake extract as claimed in one of claims 1 to 5.

15. The cosmetic composition as claimed in claim 14, **characterized in that** said extract is provided in the form of a solution having a concentration of between 10 and 40 g of dry matter per liter of solvent, advantageously 30 g/l.

16. The cosmetic composition as claimed in claim 15, **characterized in that** it contains between 0.5 and 10%, advantageously between 2 and 5%, by weight of said solution.

17. A method of cosmetic treatment for combating skin ageing, **characterized in that** an effective quantity of the cosmetic composition as claimed in one of claims 14 to 16 is applied to the skin.

## Patentansprüche

1. Extrakt des Kuchens des Samens der von einem Walnussbaum der Gattung Juglans hervorgebrachten Walnüsse, welcher durch einen Schritt wässeriger Mazeration des genannten Kuchens, anschließend durch einen Konzentrationsschritt erhalten werden kann.

2. Walnusssamenkuchenextrakt nach Anspruch 1, **dadurch gekennzeichnet, dass** der Konzentrationsschritt durch Umkehrosmose vollzogen wird.

3. Walnusssamenkuchenextrakt nach Anspruch 1, **dadurch gekennzeichnet, dass** die Mazeration bei 4°C durchgeführt wird.

4. Walnusssamenkuchenextrakt nach Anspruch 1, **dadurch gekennzeichnet, dass** auf den Konzentrationsschritt ein Trocknungs- oder Lyophilisationsschritt folgt, um ein Pulver zu erhalten.

5. Walnusssamenkuchenextrakt nach einem der Ansprüche 1 bis 4, **dadurch gekennzeichnet, dass** die Walnuss von einem gemeinen Walnussbaum (Juglans regia) hervorgebracht wird.

6. Walnusssamenkuchenextrakt nach einem der Ansprüche 1 bis 5 verwendet als Schutz der Hautzellen gegenüber dem UVB-induzierten oxidativen Stress.

7. Walnusssamenkuchenextrakt nach einem der Ansprüche 1 bis 5 verwendet als antiinflammatorisches Mittel.

8. Walnusssamenkuchenextrakt nach einem der Ansprüche 1 bis 5 verwendet als Anti-Aging-Mittel, um gegen die Alterung der Haut zu kämpfen.

9. Walnusssamenkuchenextrakt nach einem der Ansprüche 1 bis 5 verwendet als Stimulator für die Synthese der Proteine durch die Zellen der Epidermis.

10. Walnusssamenkuchenextrakt nach einem der Ansprüche 1 bis 5 verwendet als Stimulator für die Synthese der Proteine (und insbesondere des Kollagens und der Glykosaminoglykane) durch die Zellen der Lederhaut.

11. Walnusssamenkuchenextrakt nach einem der Ansprüche 1 bis 5 verwendet als Antikollagenasemittel.

12. Walnusssamenkuchenextrakt nach einem der Ansprüche 1 bis 5 verwendet als Antielastasemittel.

13. Walnusssamenkuchenextrakt nach einem der Ansprüche 1 bis 5 verwendet wegen seiner schützenden Wirkung gegenüber der induzierten Apoptose.

14. Kosmetische Zusammensetzung **dadurch gekennzeichnet, dass** sie ein Walnusssamenkuchenextrakt nach einem der Ansprüche 1 bis 5 enthält.

15. Kosmetische Zusammensetzung nach Anspruch 14, **dadurch gekennzeichnet, dass** der genannte Extrakt in Form einer Lösung mit einer Konzentration zwischen 10 und 40 g Trockensubstanz pro Liter Lösungsmittel, vorteilhafterweise 30 g/l, vorliegt.

16. Kosmetische Zusammensetzung nach Anspruch 15, **dadurch gekennzeichnet, dass** sie 0,5 bis 10, vorteilhafterweise 2 bis 5 Gew.% der genannten Lösung enthält.

17. Verfahren zur kosmetischen Behandlung, um gegen die Alterung der Haut zu kämpfen, **dadurch gekennzeichnet, dass** eine wirksame Menge der kosmetischen Zusammensetzung nach einem der Ansprüche 14 bis 16 auf die Haut aufgetragen wird.
